# EUROPÄISCHE PATENTSCHRIFT

(11) **EP 2 252 553 B1**
(45) Veröffentlichungstag und Bekanntmachung des Hinweises auf die Patenterteilung: **03.06.2015**
(21) Anmeldenummer: 09719038.3
(22) Anmeldetag: 06.03.2009
(51) Int. Cl.: C02F 3/12, C02F 11/04

(54) **VERFAHREN ZUR ABWASSERBEHANDLUNG UND ABWASSERBEHANDLUNGSANLAGE**
METHOD FOR WASTEWATER TREATMENT AND WASTEWATER TREATMENT SYSTEM
PROCÉDÉ ET DISPOSITIF DE TRAITEMENT DES EAUX USÉES

(30) Priorität: 12.03.2008 DE 102008013980
(43) Veröffentlichungstag der Anmeldung: 24.11.2010
(73) Patentinhaber: P.C.S. Pollution Control Service GmbH, 22143 Hamburg (DE)
(72) Erfinder: EWERT, Wolfgang, 22393 Hamburg (DE); SIEVERS, Michael, 38678 Clausthal-Zellerfeld (DE); BORMANN, Hinnerk, 38690 Vienenburg (DE)
(74) Vertreter: Gerstein, Hans Joachim
(86) Internationale Anmeldenummer: PCT/EP2009/001627
(87) Internationale Veröffentlichungsnummer: WO 2009/112208

(56) Entgegenhaltungen:
- EP-A- 1 364 915
- WO-A-93/15818
- WO-A-2005/003044
- DE-A1- 19 502 856
- FR-A- 2 843 106
- GB-A- 1 385 915

## Beschreibung

Die Erfindung betrifft ein Verfahren zur Abwasserbehandlung mit einer zweistrassigen anaeroben Primärschlammbehandlung in einem Faulbehälter und Überschussschlamm separat hiervon in einem weiteren Faulbehälter.

Die Erfindung betrifft weiterhin eine Abwasserbehandlungsanlage zur Behandlung von Schlämmen mit einem solchen Verfahren mit einem Faulbehälter für Primärschlamm und einem Hydrolysereaktor zur Hydrolysebehandlung von Überschussschlamm vor der anaeroben Behandlung von Überschussschlamm im Faulbehälter.

Auf kommunalen und teilweise auch auf industriellen Kläranlagen fallen im Wesentlichen zwei Schlammsorten an. Dies ist der Primär- oder Vorklärschlamm, nachfolgend als Primärschlamm bezeichnet, und der biologische Sekundär- oder Überschussschlamm, nachfolgend als Überschussschlamm bezeichnet. Nach der heutigen Verfahrensweise kommen die chemischen Schlämme kaum noch separat vor, sondern sind durch eine simultane Fällung im biologischen Schlamm integriert.

Der Primärschlamm entsteht in der Regel durch reine Schwerkraftsedimentation in Absetzbecken und kann im Anaerob-Prozess als relativ leicht abbaubare und als gut entwässerbare Komponente im gesamten Schlammsystem angesehen werden.

Der Überschussschlamm besteht weitgehend aus Bakterienmasse, die im Wesentlichen in der aeroben biologischen Behandlungsstufe entstanden ist und im Vergleich zum Primärschlamm relativ schwer anaerob abbaubar ist. Bedingt durch das starke kolloidale System und die exopolymeren Substanzen besitzt der Überschussschlamm ein hohes Wasserbindevermögen und gilt als diejenige Komponente im Faulschlammsystem, die den Entwässerungsgrad je nach Anteil stark herabsetzt.

Bei der klassischen Klärtechnik werden die beiden Schlammsorten, Primärschlamm und Überschussschlamm, gemeinsam der anaeroben Abbaustufe (unter Luftabschluss) zugeführt und entweder mesophil bei 35 bis 40 °C oder thermophil bei 50 bis 58 °C ausgefault. Die Mischung dieser Schlämme beinhaltet zwei wesentliche Nachteile:
1. Der an sich gute entwässerbare und gut abbaubare Primärschlamm wird durch einen Überschussschlamm-Anteil in seinen Eigenschaften negativ beeinflusst, was zu einer schlechteren Abbaubarkeit und einer Verringerung der Kapazität bei der Methanbildung führt.
2. Andererseits ist der Überschussschlamm im Sinne der Nährstoffnutzung ein Wertstoffträger in Bezug auf die Möglichkeit, Kohlenstoff als Methan zu nutzen und den im Überschussschlamm zu 90 % beinhalteten Phosphor als Düngemittel wieder zu gewinnen. Das Ziel, Phosphor aus dem Überschussschlamm gezielt zurück zu gewinnen, kann nur dann wirtschaftlich und sinnvoll erreicht werden, wenn die Phosphorelimination in der vorherigen Klärtechnik nach dem sogenannten BioP-Verfahren durchgeführt wird. Bei diesem Verfahren werden keine chemischen Fällmittel zur Phosphatfällung eingesetzt, sondern es wird die Eigenschaft der Bakterienmasse genutzt, unter bestimmten Prozessbedingungen vermehrt Polyphosphate anzureichern. Unter anaeroben Bedingungen werden diese als Orthophosphat wieder abgegeben (Remobilisierung). Diese natürliche Art, die Phosphate aus dem Abwasser auszuschleusen, eröffnet wirtschaftliche Möglichkeiten einer Phosphatrückgewinnung in der Klärtechnik. Die Phosphate sind aber zu ca. 90 % nur im Überschussschlamm enthalten, während der Primärschlamm nur die ca. 10 bis 15 % Restphosphate in einer anderen Form beinhaltet.

Bei der anaeroben Schlammumsetzung in den so genannten Faulbehältern werden die kohlenstoffhaltigen Verbindungen zu Methan (CH4), Kohlenstoffdioxid (CO2), Ammonium (NH4) und Wasser umgesetzt, wobei das Methan als wertvoller Energie-träger gilt und zumeist in Blockheizkraftwerken zur Erzeugung von Energie und Wärme genutzt wird. Ein Ziel bei der Optimierung im Bereich der Schlammbehandlung ist die weitestgehende Umsetzung der Kohlenstoffverbindung, um eine maximale Massenreduktion des Schlammes zu erreichen und eine maximale Energieausbeute zu erzeugen.

Nach Abschluss der anaeroben Umsetzung des Gemischs aus Primär- und Überschussschlamm wird bislang nach ca. 18 bis 30 Tagen der so genannte Faulschlamm unter Verwendung von Flockungshilfsmitteln und Entwässerungsmaschinen (Zentrifugen, Siebbandpressen, Kammerfilterpressen etc.) soweit wie möglich vom Wasser befreit, da der entwässerte Klärschlamm zumeist thermisch oder landwirtschaftlich genutzt wird: Dies stellt einen erheblichen Kostenfaktor für die Kommunen dar. Zur Kostenreduktion wird also angestrebt, die Schlämme weitestgehend aufzukonzentrieren.

Zur Erhöhung des anaeroben Abbaugrades des Überschussschlamms sind Methoden zur Vorbehandlung bekannt. Diese mechanischen, thermischen oder auch chemischen Methoden, mit denen der Überschussschlamm gewissermaßen aufgeschlossen wird, werden Desintegrationsverfahren genannt, die auch in Kombinationen eingesetzt werden können. Diese werden mit dem Ziel eingesetzt, die Bakteri-emasse soweit mechanisch, thermisch oder auch chemisch aufzuschließen, dass Substrate den anaeroben Bakterien möglichst vollständig zugänglich sind und somit die Umsatzrate und die Umsatzgeschwindigkeit wesentlich erhöht werden. Die Desintegrationsverfahren werden heutzutage im Wesentlichen beim Überschussschlamm eingesetzt, wobei nach der Anwendung des Desintegrationsverfahrens beim Überschussschlamm dieser in der Regel mit dem Primärschlamm wieder vermischt und gemeinsam dem anaeroben Prozess zugeführt wird.

Die Desintegration von Klärschlamm ist beispielsweise in N. Dichtl, J. Müller, E. Englmann, F.W. Günthert, M. Osswald: "Desintegration von Klärschlamm - ein aktueller Überblick", in: Korrespondenz Abwasser 1997 (44), Nr. 10, Seiten 1726 bis 1739 beschrieben. Es wird ausgeführt, dass bei der Desintegration die Zellwände der Mikroorganismen im Klärschlamm zerstört und die Zellinhalte freigesetzt werden. Dadurch kann der Klärschlamm besser aerob oder anaerob stabilisiert werden, so dass sowohl eine Verringerung der Schlammmenge und der Faulzeit als auch eine Erhöhung des Faulgasanfalls erreicht werden kann. Der rein mechanische Aufschluss von Überschussschlämmen führt nur bedingt zu einem beschleunigten und weitergehenden Abbau der organischen Substanz in der Faulung.

Aus DE 199 40 994 A1 ist weiterhin bekannt, den vorverfaulten Rohschlamm mit einem Dekanter aufzukonzentrieren, so dass die Feststoffphase des Faulschlamms von einer Flüssigkeitsphase getrennt wird.

DE 10 2004 023 171 A1 offenbart ein Verfahren zur Abwasserbehandlung, bei dem die biologische Abwasserbehandlung ohne Rückführung des behandelten Überschlussschlamms in dem Primärschlamm erfolgt. Der Überschussschlamm wird vom Primärschlamm in einer Klärvorrichtung nach der biologischen Behandlung abgezogen.

Durch die getrennte Behandlung des Überschussschlamms mit Ozon und einer anschließenden aeroben Oxidation in einem geeigneten Bioreaktor können die Reaktoren verkleinert und der Energieverbrauch verringert werden.

Weiterhin ist aus EP 0 784 504 B1 ein Verfahren zur Hydrolyse von organischen Materialien bekannt. Durch die Hydrolyse-Vorbehandlung von Klärschlamm wird eine Zersetzung von organischem Material in kleinere Teilchen erzielt, so dass ein hoher Gehalt von Trockensubstanz aus dem Schlamm gezogen werden kann. Die Nährstoffe sind durch die Hydrolyse-Behandlung für Bakterien zudem leichter zugänglich und der Schlamm wird pasteurisiert bzw. hygienisiert.

WO 93/15818 offenbart ein Abwasserbehandlungsverfahren, bei dem Primärschlamm einer anaeroben Behandlung zugeführt wird. Aerob behandelter Überschussschlamm wird nach der Vorklärung in mehreren Tanks in Teilströmen entwässert. Dabei wird der Schlamm aaerob durch Zufuhr von Sauerstoff behandelt. Eine anaerobe Behandlung des Überschussschlamms erfolgt nicht.

EP 1 364 915 A1 offenbart ein Abwasserbehandlungsverfahren, bei dem eine anaerobe Behandlung von Rohwasser und eine nachfolgende aerobe Behandlung erfolgt. Das Abwasser wird anschließend in einen Absetztank überführt, aus dem abgesetzter Schlamm abzogen und einer Phosphorentzugsbehandlung unterzogen wird.

FR 2 843 106 A beschreibt ein Abwasserbehandlungsverfahren bei dem eine thermische Hydrolyse von Faul-/Überschussschlamm erfolgt, das einem Klärbecken entnommen wird. Nach Separation und Aufschluss wird der aufgeschlossene hydrolysierte Überschussschlamm einer biologischen Behandlung, d. h. einer aaeroben Behandlung zugeführt.

Ausgehend hiervon ist es Aufgabe der vorliegenden Erfindung, ein verbessertes Verfahren zur Abwasserbehandlung und eine Abwasserbehandlungsanlage hierzu zu schaffen mit dem Ziel, die Energieausbeute zu erhöhen und die Rückgewinnung von Wertstoffen, wie beispielsweise Phosphor, zu verbessern. Weiterhin soll die gute Entwässerungseigenschaft des Primärschlammes verbessert werden.

Die Aufgabe wird mit dem Verfahren der eingangs genannten Art dadurch gelöst, dass der Überschussschlamm vor der anaeroben Behandlung durch eine Hydrolyse-Behandlung aufgeschlossen und somit verflüssigt und unabhängig von dem Primärschlamm einer separaten anaeroben Behandlung unterzogen wird.

Die anaerobe Behandlung des Primärschlamms findet somit getrennt von der anaeroben Behandlung des abgezogenen Überschussschlamms statt. Durch die vorgelagerte Hydrolyse-Behandlung des Überschussschlamms wird erreicht, dass der pastöse Überschussschlamm verflüssigt und die im Überschussschlamm enthaltenen wasserbindenden Gelsubstanzen durch die Erhitzung zerstört werden.

Bei den üblichen Faulzeiten von 20 Tagen sind keine Unterschiede in der Faulgasproduktion zwischen einer gemeinsamen und einer separaten anaeroben Behandlung von Überschussschlamm und Primärschlamm festzustellen.

Überraschenderweise hat sich nun gezeigt, dass eine getrennte Behandlung von hydrolysiertem Überschussschlamm einerseits und Primärschlamm andererseits insbesondere bei Faulzeiten unter 6 Tagen eine höhere Faulgas- bzw. Methanproduktion zur Folge hat, als die allgemein übliche gemeinsame anaerobe Behandlung von hydrolysiertem Überschussschlamm und Primärschlamm.

Durch die getrennte Prozessführung der beiden maßgeblichen Schlammarten, d.h. des Primärschlamms und des Überschussschlamms, kann somit wesentlich besser auf die individuellen Zielsetzungen und die unterschiedlichen Charakteristika der Schlammsorten Einfluss genommen werden. Damit ist es möglich, die Prozesse wesentlich wirtschaftlicher und technisch sinnvoller zu gestalten. Im Ergebnis können die zu entsorgenden Schlammmengen wesentlich verringert und die Ausbeute an Methan und somit Energie wesentlich um mindestens 25 bis 40 % erhöht werden. Auch die Phosphatnutzung ist wesentlich wirtschaftlicher und auch in chemisch reinerer Form zu bewerkstelligen.

Der reine Primärschlamm kann mit geringen Einsatzmengen (5 bis 8 kg/Tonne TR-Flockungshilfsmitteln) bis auf 30 bis 40 % Trockenrückstand TR entwässert werden. Der reine ausgefaulte Überschussschlamm würde sich hingegen nur bis auf 16 bis 24 % entwässern lassen. Während die herkömmlichen Gemische dieser beiden Schlämme einen Entwässerungsgrad im Bereich von 20 bis 25 % Trockenrückstand aufweisen, kann durch die vorgeschlagene separate anaerobe Behandlung von Primärschlamm und Überschussschlamm erreicht werden, dass der Überschussschlamm keine störende Komponente sowohl im Faul- als auch im Entwässerungsprozess mehr ist.

Allerdings gelingt es erst durch die vorgeschaltete Hydrolyse-Behandlung, den Überschussschlamm so aufzuarbeiten und dabei die stark wasserbindende Hydrogelstruktur zu zerstören, dass der Überschussschlamm separat und effizient in einem nachgelagerten anaeroben Behandlungsschritt abgebaut werden kann.

Im Ergebnis wird durch die separate anaerobe Behandlung von Primärschlamm und des hydrolysierten Überschussschlamms erreicht, dass die Methanausbeute und der Abbaugrad wesentlich erhöht ist.

Die Hydrolyse-Behandlung des Überschussschlamms erfolgt vorzugsweise thermisch. Dabei kann eine Erhitzung des Überschussschlamms auf einer Temperatur im Bereich von 130 bis 180 °C erfolgen. Nach dem Aufschließen des erhitzten Überschussschlamms, die durch Verweilen des Überschussschlamms in einem Druckreaktor unter der Wärmeeinwirkung für beispielsweise ca. 15 bis 45 Minuten erfolgt, erfolgt eine Entspannung des erhitzten Überschussschlamms, die für eine Zerstörung der Bakterienzellen und damit für einen weiteren mechanischen Aufschluss sorgt.

Die Hydrolyse kann durch Dampfinjektion in den Überschussschlamm, über Wärmetauscher oder mit anderen Energieträgern durchgeführt werden.

Besonders vorteilhaft ist es, wenn vor der Hydrolyse-Behandlung eine Homogenisierung des Überschussschlamms beispielsweise mit einem Homogenisator mit einem Druck von 40 bis 500 bar erfolgt. Durch die Homogenisierung wird die Effizienz des nachfolgenden Hydrolyseverfahrens aufgrund der homogenen Verteilung der Komponenten im Überschussschlamm erreicht.

Nach der Hydrolyse-Behandlung wird der hydrolysierte Überschussschlamm vorzugsweise auf eine Temperatur im Bereich von 30 bis 55 °C, besonders bevorzugt im Bereich von 35 bis 38 °C abgekühlt. Der auf diese Temperatur abgekühlte Überschussschlamm wird dann der separaten anaeroben Behandlung zugeführt.

Die anaerobe Behandlung findet besonders bevorzugt in einem Festbett- oder Schwebebettreaktor statt, wie beispielsweise einem Pelletreaktor. Derartige Festbett- oder Schwebebettreaktoren sind aus der Lebensmitteltechnologie bekannt. Diese Art von Reaktoren konnten bisher nur bei sehr niedrig viskosen, wasserähnlichen Abwässern oder Prozesswässern eingesetzt werden, da sie unter anderem sehr wesentlich von der Mobilität der Pellets (Schweben und Herumwirbeln) im Reaktor abhängig sind. Der klassische Überschussschlamm liegt in eingedickter Form aber eher pastös vor, so dass bei einem Durchströmen des Pelletreaktors die Pellets bedingt durch die hohe Viskosität des zu behandelnden Schlammmediums ausgetragen werden würden und nicht im Reaktor gehalten werden könnten.

Durch die vorgeschaltete Hydrolyse wird unter anderem auch die Gelstruktur und somit die Viskosität des Schlamms zerstört, so dass diese so stark reduziert ist, dass der Überschussschlamm bei der vorliegenden Konzentration in einem Pelletreaktor werden kann.

Damit kann eine hohe Kohlenstoffumsatzrate und eine hohe Umsatzgeschwindigkeit erreicht werden. Grund hierzu ist der Aufschluss des Überschussschlamms mit der thermischen Hydrolyse und die Ausnutzung der spezifischen hohen Umsatzraten eines Pelletreaktors, welche im Gegensatz zu einem volldurchmischten klassischen Anaerob-Reaktor nur Aufenthaltszeiten zwischen 5 und 20 Stunden benötigt, um ei-ne Kohlenstoffumsatzrate zu Methan zu erzielen, die in einem klassischen Reaktor im Schnitt 20 Tage benötigen würde.

Durch die Beschickung des Festbett- oder Schwebebettreaktors ausschließlich mit dem hydrolysierten Überschussschlamm kann sich die Bakterienmasse wesentlich besser an das einheitliche Substrat adaptieren, da eine höhere Spezialisierung in Richtung der methanbildenden Bakterien stattfindet (Vermeidung der biologischen Konkurrenzsituation). Der geschwindigkeitsbestimmende Schritt der biologischen Hydrolyse fällt hierbei weg, da das Substrat bereits hydrolysiert vorliegt, so dass die anaerobe Umsetzung begünstigt wird.

Der anaerob behandelte Überschussschlamm kann anschließend gefiltert, zentrifugiert und/oder ausgefällt werden, um Feststoffe zu extrahieren und/oder Rohstoffe, wie beispielsweise Phosphate, zurück zu gewinnen. Durch die separate Behandlung des Überschussschlamms ist die Feststoffextraktion und Rohstoffrückgewinnung wesentlich wirtschaftlicher und chemisch reiner zu bewerkstelligen. Grund ist wiederum die durch die Trennung von Überschussschlamm und Primärschlamm erreichte Einheitlichkeit der Substanz und Verbesserung des Aufschlusses.

Die extrahierten Feststoffe können dem Primärschlamm vor oder während der separaten anaeroben Behandlung des Primärschlamms zugeführt und zusammen mit dem Primärschlamm weiter abgebaut werden. Diese Feststoffe beeinträchtigen die Qualität des Primärschlamms nicht. Sie führen vielmehr zu einer Aufkonzentration und damit zu einer verbesserten Abbaubarkeit des Primärschlamms im Gegensatz zu dem hydrogelartigen, wasserbindenden Überschussschlamm.

Die bei der Hydrolyse des Überschussschlamms beispielsweise im Entspannungs-vorgang anfallende Wärmeenergie kann im Prozess weitergenutzt werden. Besonders vorteilhaft ist es, wenn die Wärmeenergie zur Hygienisierung des Primärschlamms eingesetzt wird, bevor der Primärschlamm anaerob behandelt wird. Damit können die Hygienisierungsanforderungen an den Primärschlamm mit der Wärme aus dem Prozess zur Behandlung des Überschussschlamms erfüllt werden.

Die Aufgabe wird weiterhin durch die Abwasserbehandlungsanlage der eingangs genannten Art dadurch gelöst, dass ein separater, mit dem Ausgang des Hydrolysereaktors direkt oder direkt kommunizierend derart verbundener Reaktor zur anaeroben Behandlung des hydrolysierten Überschussschlamms vorgesehen ist, dass der im Hydrolysereaktor aufgeschlossene und verflüssigte Überschussschlamm unabhängig von dem Primärschlamm einer separaten anaeroben Behandlung unterzogen wird.

Bevorzugte Ausführungsformen sind in den Unteransprüchen beschrieben.

Die Erfindung wird nachfolgend anhand eines Ausführungsbeispiels mit der beigefügten Zeichnung näher erläutert. Es zeigt:
- Figur 1: - Diagramm der Verfaulung von Überschussschlamm im Vergleich zur Verfaulung von hydrolysiertem Überschussschlamm über eine mittlere Fauldauer;
- Figur 2: - Diagramm der gemeinsamen Verfaulung von Primär- und Überschussschlamm im Vergleich zur separaten Verfaulung von Primärschlamm und hydrolysiertem Überschussschlamm über eine mittlere Fauldauer;
- Figur 3: - Diagramm der Mehrgasproduktion von hydrolysiertem Überschussschlamm in einem Hochleistungsreaktor mit fixierter Biomasse über eine mittlere Fauldauer;
- Figur 4: - Skizze einer Abwasserbehandlungsanlage mit Flussdiagramm der Stoffströme.

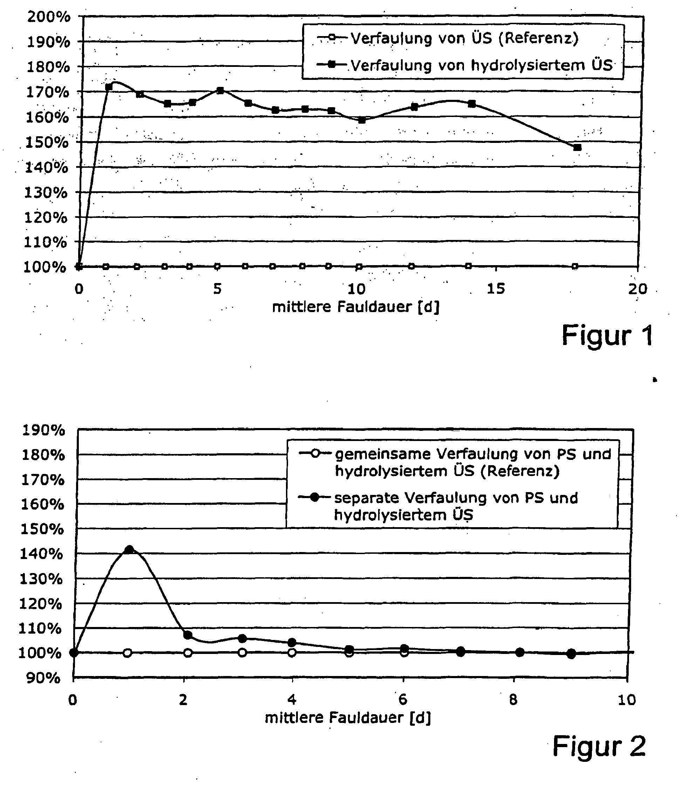

Figur 1 verdeutlicht beispielhaft die Erhöhung der Faulgas- bzw. Methanproduktion aufgrund einer thermischen Hydrolyse für den reinen Überschussschlamm in Abhängigkeit der Faulzeit. Man erkennt eine ca. 70 %ige Zunahme der Gesamt-Faulgas- bzw. Gesamt-MethanProduktion und die anschließende leichte Abnahme der Gesamt-Methanproduktion für den hydrolysierten Überschussschlamm. Bei einer ausreichend langen Faulzeit sollte dann für beide Schlämme nach allgemeiner Theorie ungefähr gleich viel Faulgas bzw. Methan produziert werden. In der Praxis wird dies jedoch in der Regel nicht erreicht.

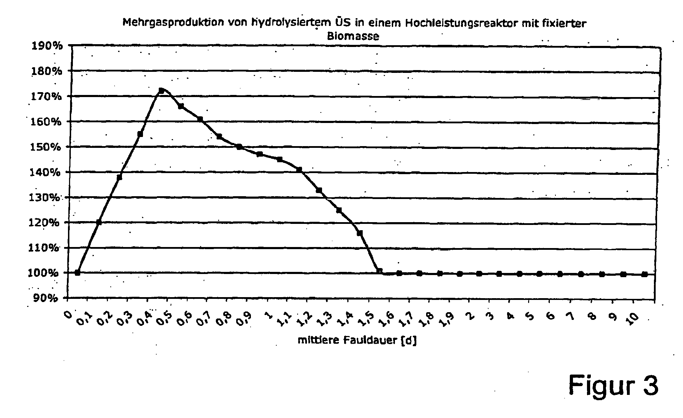

Figur 2 zeigt das Ergebnis einer gemeinsamen anaeroben Behandlung (Referenz) von hydrolysiertem Überschussschlamm und Primärschlamm im Vergleich mit dem Ergebnis einer separaten anaeroben Umsetzung in einem konventionellen auf Kläranlagen üblichem Faulbehälter von hydrolysiertem Überschussschlamm einerseits und Primärschlamm andererseits, wobei die Faulgasproduktion der beiden separaten Faulstufen addiert wurde. Es hat sich überraschenderweise gezeigt, dass nicht nur die bereits bekannte 50 bis 70 %ige Erhöhung der Faulgasproduktion des Überschussschlammanteils durch thermische Hydrolyse möglich ist, sondern zusätzlich eine noch weitere Steigerung um bis ca. 40 % insbesondere bei sehr kleinen Faulzeiten kleiner 2 Tagen aufgrund einer separaten anaeroben Behandlung von reinem Überschussschlamm einerseits und Primärschlamm andererseits. Bei Einsatz eines so genannten anaeroben Hochleistungsreaktors (Festbett oder Pelletreaktor) wird der Effekt der Mehrgasproduktion bei getrennter Behandlung des hydrolysierten Überschussschlammes im Bezug auf die Gasausbeute als auch im Bezug auf die Umsatzgeschwindigkeit erheblich gesteigert. Bei der Berechnung der Energieausbeute ist der wesentlich höhere Methangehalt bei diesen Reaktortypen zu berücksichtigen. Ein Beispiel wird in Figur 3 gezeigt. Damit wird deutlich, dass eine getrennte Behandlung von hydrolysiertem Überschussschlamm einerseits und Primärschlamm andererseits insbesondere bei Faulzeiten unter 6 Tagen eine höhere Faulgas- bzw. Methanproduktion zur Folge hat, als die allgemein übliche gemeinsame anaerobe Behandlung von hydrolysiertem Überschussschlamm und Primärschlamm.

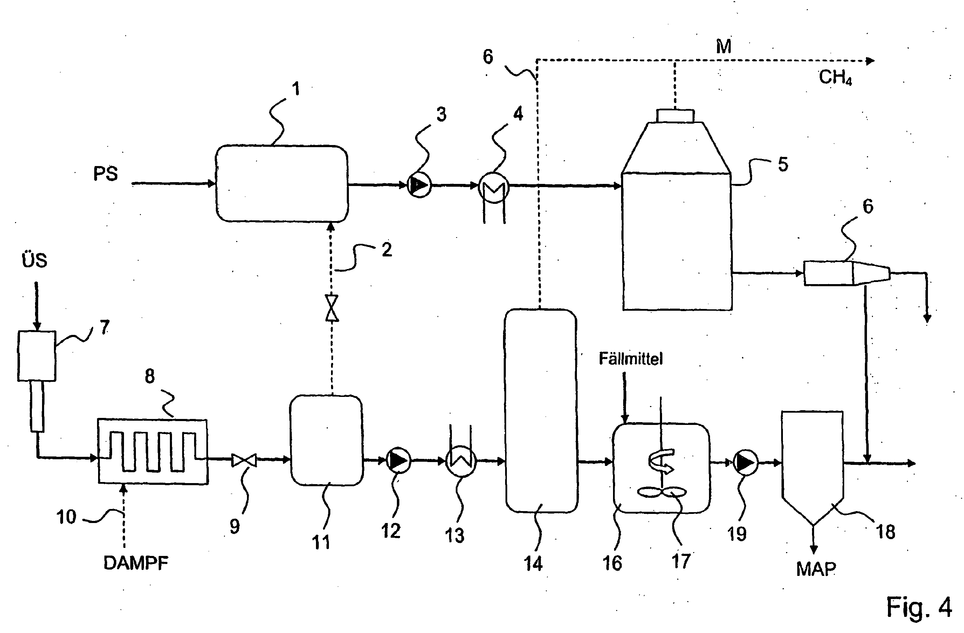

Figur 4 lässt eine schematische Skizze einer Abwasserbehandlungsanlage mit den durch die einzelnen Einrichtungen fließenden Stoffströmen erkennen.

Primärschlamm PS wird einem Hygienisierungsbehälter 1 zugeführt, der in an sich bekannter Weise zur Erwärmung und Hygienisierung des für eine Hygienisierungszeit in dem Hygienisierungsbehälter 1 gehaltenen Primärschlamms PS eingerichtet ist. Die Erwärmung erfolgt durch Zuführung von Wärmeenergie über eine Leitung 2 für ein Wärmeleitungsmedium, wie beispielsweise Dampf oder Wasser. Nach der Hygienisierung des Primärschlamms PS in dem Hygienisierungsbehälter 1 wird dieser über eine Pumpe 3 und einen Wärmetauscher 4 in einen Faulbehälter 5 zur anaeroben Behandlung geführt. In dem Faulbehälter 5 wird der Primärschlamm PS ausgefault, wobei Methangas M entsteht, das zur weiteren Verwertung beispielsweise in einem Blockheizkraftwerk (nicht dargestellt) abgeführt werden kann.

Nach der Ausfaulung wird der aus dem Primärschlamm PS entstandene Faulschlamm FS abgezogen und mit einem Entwässerungsaggregat 6, wie z.B. mit einem Flockungsreaktors mit Hilfe von Flockungsmitteln, entwässert.

In einem separaten Verarbeitungsstrang wird Überschussschlamm ÜS zunächst in einem Homogenisator 7 bei einem Druck von 40 bis 500 bar homogenisiert. Der Überschussschlamm ÜS wurde vorher vom Primärschlamm PS abgezogen und auf ca. 6 bis 12 % Trockenrückstand TR eingedickt. Anschließend wird der homogenisierte Überschussschlamm ÜS einem Hydrolysereaktor 8 zur Hydrolysebehandlung zugeführt. Der Hydrolysereaktor 8 wird der Überschussschlamm ÜS für eine Dauer von ca. 15 bis 45 Minuten bei einer Temperatur im Bereich von 130 bis 180 °C aufgeschlossen. Ein Druckhalteventil 9 sorgt für die Einhaltung der Druck- und Temperaturbedingungen. Die Beheizung des Hydrolysereaktors 8 kann wie dargestellt mit Dampf vorgenommen werden, in dem Dampf direkt über eine Dampfleitung 10 in den Überschussschlamm ÜS injiziert wird. Der Wärmeeintrag kann aber auch über Wärmetauscher oder mit Hilfe anderer Energieträger durchgeführt werden.

Nach dem Aufschließen des Überschussschlamms ÜS im Hydrolyse-(Druck)-Reaktor 8 wird durch Öffnen des Druckhalteventils 9 der Überschussschlamm ÜS schlagartig in einen Entspannungstank 11 entspannt. Dies sorgt für eine Zerstörung der Bakterienzellen und hat einen weiteren mechanischen Aufschluss des Überschussschlamms ÜS zur Folge. Im Entspannungstank 11 liegt der Überschussschlamm ÜS nun mit einer Temperatur von ca. 90 bis 100 °C vor. Über eine Pumpe 12 wird der Überschussschlamm ÜS dann einem Wärmetauscher 13 zugeführt, indem der Überschussschlamm ÜS auf die gewünschte Anaerob-Reaktortemperatur von vorzugsweise 35 bis 38 °C abgekühlt wird.

Die bei der Entspannung und bei dem Wärmetauscher anfallenden überschüssigen Energien können an anderer Stelle des Prozesses wieder sinnvoll eingesetzt werden.

So ist es vorteilhaft, wenn die in den Hygienisierungsbehälter 1 führende Leitung 2 mit dem Entspannungstank 11 verbunden ist, um das über die Leitung 2 zum Hygienisierungsbehälter 1 transportierte Wärmemedium im Entspannungstank 11 aufzuheizen. Alternativ oder zusätzlich kann das in der Leitung 2 transportierte Wärmeleitungsmedium auch über den Wärmetauscher 13 aufgeheizt werden.

Der abgekühlte hydrolysierte Überschussschlamm ÜS wird einem Reaktor 14 zur anaeroben Behandlung des Überschussschlamms ÜS separat von dem Primärschlamm PS geleitet. Dieser Anaerob-Reaktor 14 ist nicht wie in der klassischen kommunalen Klärtechnik ein volldurchmischter Reaktor, in dem die Bakterienmasse nicht suspendiert vorliegt, sondern als Fest- bzw. Schwebebettreaktor in der Form eines Pelletreaktors ausgeführt, bei dem die Bakterienmasse gewissermaßen fixiert vorliegt. Diese Pelletreaktoren sind aus der Industrie, z.B. Papier- oder Lebensmittelindustrie bekannt und dort im Einsatz.

Während der klassische Überschussschlamm ÜS in eingedickter Form pastös vorliegt, wird durch die vorgeschaltete Hydrolyse unter anderem auch die Gelstruktur zerstört und somit die Viskosität des Überschussschlamms ÜS herabgesetzt, so dass der hydrolysierte Überschussschlamm ÜS nunmehr in dem Anaerob-Reaktor 14 behandelt werden kann. Die Behandlungszeit beträgt weniger als 5 bis 6 Tage, vorzugsweise etwa 5 bis 20 Stunden, wobei die während der anaeroben Behandlung gebildeten Methanströme über eine Methangasleitung 15 zur weiteren Verwertung zusammen mit dem Methangas M aus dem Faulbehälter 5 für den Primärschlamm PS abgeführt wird. Das Methangas M sollte wie üblich gereinigt, entschwefelt, entfeuchtet und anschließend einem Gasvorratsbehälter zugeführt werden.

Nach dem Abschluss des Anaerob-Prozesses im Anaerob-Reaktor 14 liegt nun eine im Kohlenstoffgehalt sehr reduzierte, niedrig viskose Masse vor, die nur sehr wenige Feststoffe enthält und sehr gut weiter verarbeitet werden kann. Je nach Feststoffgehalt und weiterer Zielsetzung kann vor dem Eintritt und nach dem Austritt des hydrolysierten bzw. ausgefaulten Überschussschlamms ÜS in/aus dem Anaerob-Reaktor 14 der noch vorhandene Feststoff durch geeignete Filter und Zentrifugen abgetrennt werden, so dass das verbleibende feststoffarme Wasser hervorragend für eine Rückgewinnung von Rohstoffen, insbesondere von Phosphaten oder auch stickstoffhaltigen Produkten geeignet ist.

Die Rückgewinnung der Phosphate kann auf verschiedene Arten durchgeführt werden. Vorzugsweise ist eine Fällungseinheit 16 mit einem Rührwerk 17 an dem Ausgang des Anaerob-Reaktors 14 angeschlossen, die z. B. zur Fällung von Magnesium-Ammonium-Phosphat MAP aus dem aufbereiteten Überschussschlamm ÜS durch Zugabe von Magnesiumsalzen, wie z.B. Magnesiumchlorid und Einstellung eines passenden pH-Wertes von 7,5 bis 7,8 eingerichtet ist. Das schwer lösliche Magnesium-Ammonium-Phosphat MAP fällt in der Fällungseinheit 16 aus und kann in einem nachfolgenden Sedimentationsbehälter 18, der über eine Pumpe 19 an die Fällungseinheit 16 angeschlossen ist, sedimentiert und extrahiert werden. Das aus dem Sedimentationsbehälter 18 abgeleitete Wasser ist nunmehr kohlenstoff- und phosphorarm. Es kann getrennt oder zusammen mit dem durch die Entwässerungseinheit 6 abgetrennten Wassers aus dem Primärschlamm beispielsweise zur Elimination von Stickstoff N weiter behandelt werden. Diese Maßnahmen sind an sich bekannt.

Die nach dem Anaerob-Reaktor 14 gegebenenfalls abgetrennten Feststoffe sind, bedingt durch die vorgeschaltete Hydrolyse, sehr weitgehend befreit von wasserbindenden Gelsubstanzen und können dem klassischen Faulbehälter 5 für den Primärschlamm PS gefahrlos zugesetzt werden, ohne hierbei die negativen Effekte einer erschwerten Schlammentwässerung hervorzurufen. Gleichzeitig würden die noch umsetzbaren Kohlenstoffverbindungen gemeinsam mit dem Primärschlamm PS zu Methan verwandelt.

Durch die separate anaerobe Behandlung des Überschussschlamms ÜS mit Hilfe der vorgeschalteten Hydrolyse gelingt es, nicht nur den Überschussschlamm ÜS wie bislang bekannt durch Ozonzusetzung zu reduzieren, sondern den Überschussschlamm ÜS effizienter für die Energie- und Rohstoffgewinnung zu nutzen.

Die vorgeschaltete Hydrolyse hat den weiteren Effekt, dass die bislang im Faulbehälter 5 und dem anschließenden Entwässerungsaggregat 6 auftretende Kristallisation der Phosphate verhindert wird. Die Phosphate sind nämlich im Wesentlichen im Überschussschlamm ÜS vorhanden, der separat behandelt wird, wobei der Anaerob-Reaktor 14 für den Überschussschlamm ÜS einen geringeren pH-Werte aufweist, als der im Faulbehälter 5 für den Primärschlamm PS vorliegende pH-Wert.

Im Anaerob-Reaktor 14 für den Überschussschlamm ÜS kann immobilisierte Biomasse aufgrund der homogenen Struktur des hydrolysierten Überschussschlammes ÜS eingesetzt werden, was zu einer höheren Umsatzrate und Geschwindigkeit bei der anaeroben Behandlung führt. Zur anaeroben Behandlung des Primärschlamms PS, der eine Suspension darstellt, wird hingegen suspendierte Biomasse eingesetzt, was eine geringer Umsatzrate und eine längere Behandlungsdauer zur Folge hat.

## Patentansprüche

1. Verfahren zur Abwasserbehandlung mit einer anaeroben Behandlung von Primärschlamm (PS) in einem Faulbehälter (5) und einer getrennten Behandlung von Überschussschlamm (ÜS), **dadurch gekennzeichnet, dass** der Überschussschlamm (ÜS) in einer Hydrolysebehandlung aufgeschlossen und verflüssigt und unabhängig von dem Primärschlamm (PS) einer separaten anaeraben Behandlung unterzogen wird.

2. Verfahren nach Anspruch 1, **gekennzeichnet durch** thermische und/oder chemische Hydrolyse des Überschussschlamms (ÜS).

3. Verfahren nach Anspruch 2, **dadurch gekennzeichnet, dass** die thermische Hydrolyse durch Erhitzen des Überschussschlamms (ÜS) auf eine Temperatur im Bereich von 130 bis 180 °C erfolgt und ein Aufschließen des erhitzten Überschussschlamms (ÜS) in einem Hydrolysereaktor (8) und Entspannung des erhitzten Überschussschlamms (ÜS) nach einer Verweilzeit in dem Hydrolysereaktor (8) vorgesehen ist.

4. Verfahren nach Anspruch 3, **dadurch gekennzeichnet, dass** die Verweilzeit des Überschussschlamms (ÜS) im Hydrolysereaktor (8) zum Aufschluss von Zellen 15 bis 60 Minuten und bevorzugt bis 45 Minuten beträgt.

5. Verfahren nach einem der vorhergehenden Ansprüche, **gekennzeichnet durch** Injektion von Dampf in den Überschussschlamm (ÜS) zur Hydrolysebehandlung.

6. Verfahren nach einem der vorhergehenden Ansprüche, **gekennzeichnet durch** Anhebung des pH-Wertes des Überschussschlammes (ÜS) auf Werte zwischen 9 bis 14 vorzugsweise 9 bis 12 und Erhöhung der Temperatur auf 50 bis 130 °C vorzugsweise 60 bis 100 °C für die chemisch/thermische Hydrolyse.

7. Verfahren nach einem der vorhergehenden Ansprüche, **gekennzeichnet durch** Homogenisierung des Überschussschlamms (ÜS) vor der Hydrolysebehandlung.

8. Verfahren nach einem der vorhergehenden Ansprüche, **gekennzeichnet durch** Abkühlen des hydrolisierten Überschussschlamms (ÜS) auf eine Temperatur im Bereich von 30 bis 55 °C und bevorzugt im Bereich von 35 bis 38 °C und anaerobe Behandlung des derart abgekühlten Überschussschlamms (ÜS).

9. Verfahren nach einem der vorhergehenden Ansprüche, **dadurch gekennzeichnet, dass** die anaerobe Behandlung des Überschussschlamms (ÜS) in einem Festbettreaktor oder Schwebebettreaktor (14) erfolgt.

10. Verfahren nach Anspruch 9, **dadurch gekennzeichnet, dass** der Festbettreaktor (14) ein Pelletreaktor ist.

11. Verfahren nach einem der vorhergehenden Ansprüche, **gekennzeichnet durch** Filtern, Zentrifugieren und/oder Fällen des anaerob behandelten Überschussschlamms (ÜS) zum Extrahieren von Feststoffen und/oder Rückgewinnen von Rohstoffen, beispielsweise von Phosphaten oder Stickstoffverbindungen.

12. Verfahren nach Anspruch 11, **gekennzeichnet durch** Zuführen von aus dem anaerob behandelten Überschussschlamm (ÜS) abgetrennten Feststoffen zu dem Primärschlamm (PS) vor oder während der separaten anaeroben Behandlung des Primärschlamms (PS).

13. Verfahren nach einem der vorhergehenden Ansprüche, **gekennzeichnet durch** Aufheizen des Primärschlamms (PS) mittels bei der Hydrolyse des Überschussschlamms (ÜS) anfallenden Wärmeenergie zur Hygienisierung.

14. Verfahren nach einem der vorhergehenden Ansprüche, **dadurch gekennzeichnet, dass** die anaerobe Behandlung des hydrolisierten Überschussschlamms (ÜS) für eine Dauer von kleiner fünf Tagen und vorzugsweise von 5 bis 20 Stunden durchgeführt wird.

15. Verfahren nach einem der vorhergehenden Ansprüche, **gekennzeichnet durch** Ableiten von bei der anaeroben Behandlung des Primärschlamms (PS) und der separaten anaeroben Behandlung des Überschussschlamms (ÜS) anfallenden Methangases (M) zur weiteren Verwertung.

16. Abwasserbehandlungsanlage zur Behandlung von Abwässern und/oder Schlämmen mit dem Verfahren nach einem der vorhergehenden Ansprüche mit einem Faulbehälter (5) für Primärschlamm (PS) und einem Hydrolysereaktor (8) zur Hydrolysebehandlung von Überschussschlamm (ÜS), **gekennzeichnet durch** einen separaten, mit dem Ausgang des Hydrolysereaktors (8) direkt oder indirekt kommunizierend derart verbundenen Reaktor (14) zur anaeroben Behandlung des hydrolisierten Überschussschlamms (ÜS), dass der im Hydrolysereaktor (8) aufgeschlossene und verflüssigte Überschussschlamm (ÜS) unabhängig von dem Primärschlamm (PS) einer separaten anaeroben Behandlung unterzogen wird.

17. Abwasserbehandlungsanlage nach Anspruch 16, **gekennzeichnet durch** einen dem Hydrolysereaktor (8) vorgeschalteten Homogenisator (7) zur Desintegration des Überschussschlamms (ÜS).

18. Abwasserbehandlungsanlage nach Anspruch 16, **dadurch gekennzeichnet, dass** der Reaktor (14) zur anaeroben Behandlung des hydrolisierten Überschussschlamms (ÜS) ein Festbettreaktor oder Schwebebettreaktor ist.

19. Abwasserbehandlungsanlage nach Anspruch 18, **dadurch gekennzeichnet, dass** der Festbettreaktor oder Schwebebettreaktor ein Pelletreaktor ist.

20. Abwasserbehandlungsanlage nach einem der Ansprüche 16 bis 19, **dadurch gekennzeichnet, dass** der Hydrolysereaktor (8) einen nachgeschalteten Entspannungstank (11) halt.

21. Abwasserbehandlungsanlage nach einem der Ansprüche 16 bis 20, **gekennzeichnet durch** eine stromabwärts dem Ausgang des Reaktors (14) zur anaeroben Behandlung des Überschussschlamms (ÜS) folgende Fällungseinheit (16) mit einer Zufuhr für Fällmittel zu Ausfällen von Phosphaten aus dem hydolisierten und anaerob behandelten Überschussschlamm (ÜS).

22. Abwasserbehandlungsanlage nach einen der Ansprüche 16 bis 21, **gekennzeichnet durch** eine stromabwärts dem Ausgang des Reaktors (14) zur anaeroben Behandlung des Überschussschlamms (ÜS) folgenden Zentrifuge und/ oder Filter zum Abscheiden von Feststoffen aus dem hydrolisierten und anaerob behandelten Überschussschlamm (ÜS).

23. Abwasserbehandlungsanlage nach einem der Ansprüche 16 bis 20, **gekennzeichnet durch** eine Leitung (2) für Wärmeleitungsmedium zwischen dem Hydrolysereaktor (8), vorzugsweise dem Entspannungstank (11), und einem Hygienisierungsbehälter (1) für Primärschlamm (PS), wobei der Hygienisierungsbehälter (1) eine Heizeinheit zur Erwärmung von im Hygienisierungsbehälter (1) eingeführten Primärschlamm (PS) mittels Wärmeenergie hat, die **durch** das Wärmeleitungsmedium von dem Hydrolysereaktor (8) über die Leitung (2) übertragbar ist.

24. Abwasserbehandlungsanlage nach einem der Ansprüche 16 bis 23, **gekennzeichnet durch** einen Wärmetauscher (13) zwischen Hydrolysereaktor (8) und Reaktor (14) zur anaeroben Behandlung des hydrotisierten Überschussschlamms (ÜS) zur Abkühlung des Überschussschlamms (ÜS).

## Claims

1. A method for wastewater treatment using an anaerobic treatment of primary sludge (PS) in a digestion vessel (5) and a separate treatment of excess sludge (ES), **characterized in that** the excess sludge (ES) is disrupted and liquefied in a hydrolysis treatment and, independently of the primary sludge (PS), is subjected to a separate anaerobic treatment.

2. The method as claimed in claim 1, **characterized by** thermal and/or chemical hydrolysis of the excess sludge (ES).

3. The method as claimed in claim 2, **characterized in that** the thermal hydrolysis proceeds by heating the excess sludge (ES) to a temperature in the range from 130 to 180° C, and disruption of the heated excess sludge (ES) in a hydrolysis reactor (8) and expansion of the heated excess sludge (ES) after a residence time in the hydrolysis reactor (8) are provided for.

4. The method as claimed in claim 3, **characterized in that** the residence time of the excess sludge (ES) in the hydrolysis reactor (8) for disruption of cells is 15 to 60 minutes and preferably up to 45 minutes.

5. The method as claimed in any one of the preceding claims, **characterized by** injection of steam into the excess sludge (ES) for the hydrolysis treatment.

6. The method as claimed in any one of the preceding claims, **characterized by** raising the pH of the excess sludge (ES) to values between 9 and 14, preferably 9 and 12, and increasing the temperature to 50 to 130° C, preferably 60 to 100° C, for the chemical/thermal hydrolysis.

7. The method as claimed in any one of the preceding claims, **characterized by** homogenization of the excess sludge (ES) before the hydrolysis treatment.

8. The method as claimed in any one of the preceding claims, **characterized by** cooling the hydrolyzed excess sludge (ES) to a temperature in the range from 30 to 55° C, and preferably in the range from 35 to 38° C, and anaerobic treatment of the excess sludge (ES) thus cooled.

9. The method as claimed in any one of the preceding claims, **characterized in that** the anaerobic treatment of the excess sludge (ES) proceeds in a fixed-bed reactor or fluidized-bed reactor (14).

10. The method as claimed in claim 9, **characterized in that** the fixed-bed reactor (14) is a pellet reactor.

11. The method as claimed in any one of the preceding claims, **characterized by** filtering, centrifuging and/or precipitating the anaerobically treated excess sludge (ES) for extracting solids and/or recovering raw materials, for example phosphates or nitrogen compounds.

12. The method as claimed in claim 11, **characterized by** feeding solids separated off from the anaerobically treated excess sludge (ES) to the primary sludge (PS) before or during the separate anaerobic treatment of the primary sludge (PS).

13. The method as claimed in any one of the preceding claims, **characterized by** heating the primary sludge (PS) for sanitation by means of heat energy occurring on hydrolysis of the excess sludge (ES).

14. The method as claimed in any one of the preceding claims, **characterized in that** the anaerobic treatment of the hydrolyzable excess sludge (ES) is carried out for a time of less than 5 days, and preferably of 5 to 20 hours.

15. The method as claimed in any one of the preceding claims, **characterized by** discharging, for further use, methane gas (M) occurring in the anaerobic treatment of the primary sludge (PS) and the separate anaerobic treatment of the excess sludge (ES).

16. A wastewater treatment system for treating wastewaters and/or sludges using the method as claimed in any one of the preceding claims, having a digestion vessel (5) for primary sludge (PS) and a hydrolysis reactor (8) for the hydrolysis treatment of excess sludge (ES), **characterized by** a separate reactor (14) for the anaerobic treatment of the hydrolyzed excess sludge (ES), said reactor (14) being connected so as to communicate directly or indirectly with the outlet of the hydrolysis reactor (8) such, that the excess sludge (ES) which is disrupted and liquefied in the hydrolysis reactor (8) is subjected to a separate anaerobic treatment independently of the primary sludge (PS).

17. The wastewater treatment system as claimed in claim 16, **characterized by** a homogenizer (7) for disintegrating the excess sludge (ES), said homogenizer (7) being connected upstream of the hydrolysis reactor (8).

18. The wastewater treatment system as claimed in claim 16, **characterized in that** the reactor (14) for the anaerobic treatment of the hydrolyzed excess sludge (ES) is a fixed-bed reactor or fluidized-bed reactor.

19. The wastewater treatment system as claimed in claim 18, **characterized in that** the fixed-bed reactor or fluidized-bed reactor is a pellet reactor.

20. The wastewater treatment system as claimed in any one of claims 16 to 19, **characterized in that** the hydrolysis reactor (8) has a downstream expansion tank (11).

21. The wastewater treatment system as claimed in any one of claims 16 to 19, **characterized by** a precipitation unit (16) following downstream of the outlet of the reactor (14) for the anaerobic treatment of the excess sludge (ES) and having a feed for precipitant for precipitating phosphates from the hydrolyzed and anaerobically treated excess sludge (ES).

22. The wastewater treatment system as claimed in any one of claims 16 to 21, **characterized by** a centrifuge and/or filter for separating off solids from the hydrolyzed and anaerobically treated excess sludge (ES), said centrifuge and/or filter following downstream of the outlet of the reactor (14) for the anaerobic treatment of the excess sludge (ES).

23. The wastewater treatment system as claimed in any one of claims 16 to 20, **characterized by** a line (2) for heat conduction medium between the hydrolysis reactor (8), preferably the expansion tank (11), and a sanitation vessel (1) for primary sludge (PS), wherein the sanitation vessel (1) has a heating unit for heating primary sludge (PS) by means of heat energy which can be transferred by the heat conduction medium from the hydrolysis reactor (8) via the line (2), said primary sludge (PS) being introduced in the sanitation vessel (1).

24. The wastewater treatment system as claimed in any one of claims 16 to 23, **characterized by** a heat exchanger (13) between hydrolysis reactor (8) and reactor (14) for the anaerobic treatment of the hydrolyzed excess sludge (ES) for cooling the excess sludge (ES).

## Revendications

1. Procédé de traitement d'eaux usées comprenant un traitement anaérobie d'une boue primaire (BP) dans un digesteur (5) et un traitement séparé d'une boue excédentaire (BE), **caractérisé en ce que** la boue excédentaire (BE) est décomposée et liquéfiée par un traitement d'hydrolyse, et soumise indépendamment de la boue primaire (BP) à un traitement anaérobie séparé.

2. Procédé selon la revendication 1, **caractérisé par** une hydrolyse thermique et/ou chimique de la boue excédentaire (BE).

3. Procédé selon la revendication 2, **caractérisé en ce que** l'hydrolyse thermique a lieu par chauffage de la boue excédentaire (BE) à une température dans la plage allant de 130 à 180 °C, et une décomposition de la boue excédentaire (BE) chauffée dans un réacteur d'hydrolyse (8) et une détente de la boue excédentaire (BE) chauffée après un temps de séjour dans le réacteur d'hydrolyse (8) sont prévues.

4. Procédé selon la revendication 3, **caractérisé en ce que** le temps de séjour de la boue excédentaire (BE) dans le réacteur d'hydrolyse (8) pour la décomposition de cellules est de 15 à 60 minutes et de préférence de jusqu'à 45 minutes.

5. Procédé selon l'une quelconque des revendications précédentes, **caractérisé par** une injection de vapeur dans la boue excédentaire (BE) pour le traitement d'hydrolyse.

6. Procédé selon l'une quelconque des revendications précédentes, **caractérisé par** une augmentation du pH de la boue excédentaire (BE) à des valeurs comprises entre 9 et 14, de préférence entre 9 et 12, et une augmentation de la température à 50 à 130 °C, de préférence 60 à 100 °C, pour l'hydrolyse chimique/thermique.

7. Procédé selon l'une quelconque des revendications précédentes, **caractérisé par** une homogénéisation de la boue excédentaire (BE) avant le traitement d'hydrolyse.

8. Procédé selon l'une quelconque des revendications précédentes, **caractérisé par** un refroidissement de la boue excédentaire (BE) hydrolysée à une température dans la plage allant de 30 à 55 °C et de préférence dans la plage allant de 35 à 38 °C, et un traitement anaérobie de la boue excédentaire (BE) ainsi refroidie.

9. Procédé selon l'une quelconque des revendications précédentes, **caractérisé en ce que** le traitement anaérobie de la boue excédentaire (BE) a lieu dans un réacteur à lit fixe ou un réacteur à lit flottant (14).

10. Procédé selon la revendication 9, **caractérisé en ce que** le réacteur à lit fixe (14) est un réacteur à granulés.

11. Procédé selon l'une quelconque des revendications précédentes, **caractérisé par** une filtration, une centrifugation et/ou une précipitation de la boue excédentaire (BE) traitée par voie anaérobie pour l'extraction de solides et/ou la récupération de matières premières, par exemple de phosphates ou de composés azotés.

12. Procédé selon la revendication 11, **caractérisé par** une introduction des solides séparés de la boue excédentaire (BE) traitée par voie anaérobie dans la boue primaire (BP) avant ou pendant le traitement anaérobie séparé de la boue primaire (BP).

13. Procédé selon l'une quelconque des revendications précédentes, **caractérisé par** un chauffage de la boue primaire (BP) au moyen de l'énergie calorifique formée lors de l'hydrolyse de la boue excédentaire (BE) pour la désinfection.

14. Procédé selon l'une quelconque des revendications précédentes, **caractérisé en ce que** le traitement anaérobie de la boue excédentaire (BE) hydrolysée est réalisé pendant une durée inférieure à cinq jours et de préférence de 5 à 20 heures.

15. Procédé selon l'une quelconque des revendications précédentes, **caractérisé par** le déchargement du méthane gazeux (M) formé lors du traitement anaérobie de la boue primaire (BP) et du traitement anaérobie séparé de la boue excédentaire (BE) vers une utilisation ultérieure.

16. Unité de traitement d'eaux usées pour le traitement d'eaux usées et/ou de boues par le procédé selon l'une quelconque des revendications précédentes, comprenant un digesteur (5) pour une boue primaire (BP) et un réacteur d'hydrolyse (8) pour le traitement d'hydrolyse d'une boue excédentaire (BE), **caractérisée par** un réacteur (14) séparé pour le traitement anaérobie de la boue excédentaire (BE) hydrolysée, relié en communication directe ou indirecte avec la sortie du réacteur d'hydrolyse (8), de sorte que la boue excédentaire (BE) décomposée et liquéfiée dans le réacteur d'hydrolyse (8) soit soumise indépendamment de la boue primaire (BP) à un traitement anaérobie séparé.

17. Unité de traitement d'eaux usées selon la revendication 16, **caractérisée par** un homogénéisateur (7) connecté en amont du réacteur d'hydrolyse (8), pour la désintégration de la boue excédentaire (BE).

18. Unité de traitement d'eaux usées selon la revendication 16, **caractérisée en ce que** le réacteur (14) pour le traitement anaérobie de la boue excédentaire (BE) hydrolysée est un réacteur à lit fixe ou un réacteur à lit flottant.

19. Unité de traitement d'eaux usées selon la revendication 18, **caractérisée en ce que** le réacteur à lit fixe ou le réacteur à lit flottant est un réacteur à granulés.

20. Unité de traitement d'eaux usées selon l'une quelconque des revendications 16 à 19, **caractérisée en ce qu'**une cuve de détente (11) est connectée en aval du réacteur d'hydrolyse (8).

21. Unité de traitement d'eaux usées selon l'une quelconque des revendications 16 à 20, **caractérisé par** une unité de précipitation (16) en aval de la sortie du réacteur (14) pour le traitement anaérobie de la boue excédentaire (BE), comprenant une entrée pour un agent de précipitation pour la précipitation de phosphates à partir de la boue excédentaire (BE) hydrolysée et traitée par voie anaérobie.

22. Unité de traitement d'eaux usées selon l'une quelconque des revendications 16 à 21, **caractérisée par** une centrifugeuse et/ou un filtre en aval de la sortie du réacteur (14) pour le traitement anaérobie de la boue excédentaire (BE), pour la séparation de solides à partir de la boue excédentaire (BE) hydrolysée et traitée par voie anaérobie.

23. Unité de traitement d'eaux usées selon l'une quelconque des revendications 16 à 20, **caractérisée par** une conduite (2) pour un milieu caloporteur entre le réacteur d'hydrolyse (8), de préférence la cuve de détente (11), et un contenant de désinfection (1) pour la boue primaire (BP), le contenant de désinfection (1) comprenant une unité de chauffage pour le chauffage de la boue primaire (BP) introduite dans le contenant de désinfection (1) au moyen d'une énergie calorifique qui peut être transférée par le milieu caloporteur depuis le réacteur d'hydrolyse (8) par la conduite (2).

24. Unité de traitement d'eaux usées selon l'une quelconque des revendications 16 à 23, **caractérisée par** un échangeur de chaleur (13) entre le réacteur d'hydrolyse (8) et le réacteur (14) pour le traitement anaérobie de la boue excédentaire (BE) hydrolysée, pour le refroidissement de la boue excédentaire (BE).
